(19) Europäisches Patentamt
European Patent Office
Office européen des brevets

(11) **EP 3 634 283 B1**

(12) **EUROPÄISCHE PATENTSCHRIFT**

(45) Veröffentlichungstag und Bekanntmachung des Hinweises auf die Patenterteilung:
**09.12.2020 Patentblatt 2020/50**

(21) Anmeldenummer: **18729950.8**

(22) Anmeldetag: **06.06.2018**

(51) Int Cl.:
***A61B 18/12*** *(2006.01)*

(86) Internationale Anmeldenummer:
**PCT/EP2018/064929**

(87) Internationale Veröffentlichungsnummer:
**WO 2018/224564 (13.12.2018 Gazette 2018/50)**

(54) **ELEKTROCHIRURGIE-GENERATOR**

ELECTROSURGICAL GENERATOR

GÉNÉRATEUR ÉLECTROCHIRURGICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.06.2017 DE 102017112684**

(43) Veröffentlichungstag der Anmeldung:
**15.04.2020 Patentblatt 2020/16**

(73) Patentinhaber: **Olympus Winter & Ibe GmbH 22045 Hamburg (DE)**

(72) Erfinder:
• **RAMIN, Daniel 14558 Nuthetal (DE)**
• **FÄHSING, Thomas 38889 Blankenburg (DE)**
• **SCHIDDEL, Stefan 14532 Stahnsdorf (DE)**

(74) Vertreter: **Eisenführ Speiser Patentanwälte Rechtsanwälte PartGmbB Anna-Louisa-Karsch-Strasse 2 10178 Berlin (DE)**

(56) Entgegenhaltungen:
WO-A1-98/44855    DE-A1-102007 051 097
DE-A1-102010 040 824    DE-A1-102011 078 452

**Beschreibung**

Die Erfindung betrifft einen Elektrochirurgie-Generator mit Anschlüssen zum Anschließen eines elektrochirurgischen Instruments.

Der Elektrochirurgie-Generator weist einen Hochspannungsgenerator auf, der mit den Anschlüssen für das elektrochirurgische Instrument elektrisch verbunden und ausgebildet ist, in seinem Betriebszustand einen hochfrequenten Wechselstorm zu erzeugen und über die Anschlüsse abzugeben. Der Elektrochirurgie-Generator weist eine Wirkleistungsbestimmungseinheit auf, die eine Phasenverschiebung zwischen Strom und Spannung eines von dem Elektrochirurgie-Generator im Betrieb abgegebenen, hochfrequenten Wechselstroms erfassen kann, um die im Anwendungsfall von dem Elektrochirurgie-Generator über ein angeschlossenes elektrochirurgisches Instrument abgegebene wirksame Leistung zu bestimmen.

Derartige Elektrochirurgie-Generatoren werden unter anderem in Verbindung mit solchen elektrochirurgischen Instrumenten verwendet, die eine Koagulation oder Ablation von Körpergewebe ermöglichen. Beispielsweise ist es bekannt, über im Anwendungsfall mit Körpergewebe in Kontakt stehenden Elektroden des elektrochirurgischen Instrumentes einen hochfrequenten Wechselstrom durch das anliegende Körpergewebe zu leiten, um das Körpergewebe auf diese Weise so zu erwärmen, dass es denaturiert. Auf diese Weise lassen sich beispielsweise Tumore behandeln oder Gewebeteile zu anderen Zwecken veröden oder auch zu fusionieren.

Bekannte Elektrochirurgie-Generatoren weisen eine Wirkleistungsbestimmungseinheit auf, die ausgebildet ist, ein eine Phasenverschiebung zwischen Strom und Spannung eines im Betrieb von dem Elektrochirurgie-Generator abgegebenen Wechselstroms widerspiegelndes Ausgangssignal zu erzeugen. Die Wirkleistungsbestimmungseinheit weist eine Phasenverschiebung-Bestimmungseinheit mit einem Tiefpassfilter auf und ist ausgebildet, eine zeitliche Differenz zwischen den Nulldurchgängen von Strom und Spannung zu bestimmen und ein Ausgangssignal zu bilden, dessen Betrag diese zeitliche Differenz widerspiegelt. Die Phasenverschiebung-Bestimmungseinheit umfasst vorzugsweise einen Nulldurchgangsdetektor, der ein Detektorausgangssignal erzeugt, dessen Pulsbreite genau der Dauer der Phasenverschiebung zwischen Strom und Spannung entspricht. Dieses Detektorausgangssignal wird dann von dem Tiefpassfilter in eine Gleichspannung umgewandelt, deren Betrag die Phasenverschiebung repräsentiert. Wenn auf diese Weise die Phasenverschiebung bekannt ist, kann die Wirkleistung in an sich bekannter Weise als Produkt aus den Effektivwerten von Strom und Spannung sowie dem Kosinus des Phasenverschiebungswinkels bestimmt werden.

Es hat sich herausgestellt, dass solche bekannten Wirkleistungsbestimmungseinheiten in der Praxis gelegentlich falsche Werte liefern.

In DE 10 2011 078452 A1 ist ein Elektrowerkzeug mit einem Wechselrichter zur Erzeugung einer Versorgungsspannung für ein Ultraschallgerät des Elektrowerkzeugs beschrieben.

In DE 10 2010 040824 A1 ist ein Hochfrequenz-Chirurgiegerät zum Erzeugen von einem hochfrequenten Wechselstrom beschrieben.

In WO 98/44855 A1 ist eine Einrichtung zur Überwachung der Applikation einer Neutralelektrode bei der monopolaren HF-Chirurgie mit einem Impedanzsensor beschrieben.

In DE 10 2007 051097 A1 ist ein HF-Chirurgiegerät zum Schneiden und/oder Koagulieren von biologischem Gewebe beschrieben.

Die der Erfindung zugrundeliegende Aufgabe ist es daher, einen verbesserten Elektrochirurgie-Generator zu erschaffen, der die von ihm abgegebene Wirkleistung zuverlässiger bestimmt.

Erfindungsgemäß ist hierzu bei einem Elektrochirurgie-Generator der zuvor beschriebenen Art eine Schalteinheit vorgesehen, die derart angeordnet und ausgebildet ist, dass dem Tiefpassfilter nur dann ein Detektorausgangssignal zugeführt wird, wenn sich der Hochspannungsgenerator in seinem eingeschaltetem Zustand befindet, nicht aber, wenn der Hochspannungsgenerator abgeschaltet ist und die über die Anschlüsse ggf. abgegebene Leistung auf Nachschwingungen nach dem Abschalten beruht.

Im einfachsten Fall wird beispielsweise die Wirkleistungsbestimmungseinheit sofort abgeschaltet, wenn auch der Hochspannungsgenerator des Elektrochirurgie-Generators abgeschaltet ist. Vorzugsweise ist die Schalteinheit jedoch zwischen einem Ausgang der Phasenverschiebung-Bestimmungseinheit und einem Eingang des Tiefpassfilters angeordnet, sodass der Eingang des Tiefpassfilters ein Nullsignal erhält, sobald der Hochspannungsgenerator abgeschaltet ist.

Die Erfindung schließt die Erkenntnis ein, dass Wirkleistungsbestimmungseinheiten der eingangs beschriebenen Art insbesondere durch Nachschwingen oder Ausschwingen nach dem Ausschalten des Hochspannungsgenerators derart beeinträchtigt werden, dass sie fehlerhafte Ausgangswerte liefern. Daher ist es vorteilhaft, wenn die Wirkleistungsbestimmungseinheit sofort nach Ausschalten des Hochspannungsgenerators kein unter Umständen falsches, von der Phasenverschiebung-Bestimmungseinheit generiertes Detektorausgangssignal empfängt.

Die Schalteinheit bewirkt ein hardwareseitiges Auskoppeln des Nachschwingens, so dass nur solche Signale in das Bestimmen des die Wirkleistung des abgegebenen Wechselstroms repräsentierenden Signals eingehen, die erfasst werden, wenn ein Eingangssteuersignal (das Generator-Ein-Signal) vorliegt,

In einer besonders vorteilhaften Ausführungsvariante ist die Schalteinheit als UND-Gatter mit zwei Eingängen und einem Ausgang ausgebildet, von denen einer der Eingänge mit einem Ausgang der Phasenverschiebung-Bestimmungseinheit verbunden ist und von denen der andere Eingang im Betriebszustand des Hochspannungsgenerators ein Generator-Ein-Signal empfängt, und dessen Ausgang mit dem Tiefpassfilter verbunden ist. Mit anderen Worten ist zwischen dem Ausgang der Phasenverschiebung-Bestimmungseinheit und dem Eingang des Tiefpassfilters ein UND-Gatter geschaltet, dass das Detektorausgangssignal der Phasenverschiebung-Bestimmungseinheit nur dann an den Eingang des Tiefpassfilters weitergibt, wenn am zweiten Eingang des UND-Gatters ein Generator-Ein-Signal anliegt. Das Generator-Ein-Signal ist dabei ein Signal, das nur dann anliegt, wenn der Hochspannungsgenerator im Betrieb ist und nicht ausgeschaltet ist. Falls der Hochspannungsgenerator ausgeschaltet ist, ist das Generator-Ein-Signal ein Nullsignal und das UND-Gatter sperrt, sodass das Detektorausgangssignal nicht zum Eingang des Tiefpassfilters gelangen kann.

In weiteren bevorzugten Ausführungsvarianten besitzt die Phasenverschiebung-Bestimmungseinheit zwei Komparatoren. Von diesen ist ein erster Komparator ausgebildet und angeordnet, ein Spannungssignal mit einem Nullsignal zu vergleichen und ein Ausgangssignal zu erzeugen, wenn ein Momentanwert der Spannung größer ist, als das Nullsignal. Der zweite der Kooperatoren ist ausgebildet und angeordnet, ein Nullsignal mit einem Stromsignal zu vergleichen und ein Ausgangssignal zu erzeugen, wenn das Nullsignal größer ist als ein Momentanwert des Stroms, also wenn der Momentanwert des Stroms negativ ist. Mit anderen Worten liefert der erste Komparator ein Ausgangssignal, wenn der Momentanwert der Spannung positiv, also größer als Null ist, während der zweite Komparator ein Ausgangssignal liefert, wenn der Stromwert negativ, also kleiner als Null, ist. Auf diese Weise liefern nur dann beide Komparatoren ein Ausgangssignal, wenn der Momentanwert der Spannung einen Nulldurchgang von positiv zu negativer Richtung durchlaufen hat und gleichzeitig der Momentanwert des Stroms noch keinen Nulldurchang von positiv zu negativ durchlaufen hat.

Es versteht sich, dass die Komparatoren auch genau umgekehrt geschaltet sein können, sodass der erste Komparator ein Ausgangssignal liefert, wenn der Momentanwert der Spannung kleiner ist als Null während der zweite Komparator nur dann an ein Ausgangssignal liefert, wenn der zweite Komparator nur dann an ein Ausgangsignal liefert, wenn der Momentwert des Stroms größer ist als Null ist.

Die Ausgänge der beiden Komparatoren in dieser bevorzugten Ausführungsvariante sind so miteinander verbunden, dass die beiden Komparatoren nur dann ein kombiniertes Ausgangssignal als Detektorausgangssignal mit einem über einem Schwellwert liegenden Betrag ausgeben, wenn der Momentanwert der Spannung positiv - also größer als das Nullsignal - ist und gleichzeitig der Momentanwert des Stroms negativ - also kleiner als das Nullsignal - ist. Im umgekehrten, genauso möglichen Fall sind die Ausgänge der beidem Komparatoren so mit einander verbunden, dass die beiden Komparatoren nur dann ein kombiniertes Ausgangssignal als Detektorausgangssignal über einem Schwellwert liegende Betrag ausgeben, wenn der Momentanwert des Stroms positiv - also größer als das Nullsignal - ist und gleichzeitig der Momentwert der Spannung negativ - also kleiner als das Nullsignal - ist.

Weitere bevorzugte Ausführungsvarianten des Elektrochirurgie-Generator zeichnen sich dadurch aus, dass der Hochspannungsgenerator ausgebildet ist, in seinem Betriebszustand den hochfrequenten Wechselstrom mit einem Tastverhältnis getaktet über die Anschlüsse abzugeben. Mit anderen Worten wird nicht ein kontinuierlicher Wechselstrom über die Anschlüsse abgegeben, sondern der Wechselstrom wird regelmäßig unterbrochen, also getaktet. Dies kann beispielsweise mittels einer Phasenanschnittsteuerung geschehen, sodass nicht dauerhaft die maximale Wirkleistung abgegeben zu werden braucht, sondern dass sich die abzugebende Wirkleistung auch gesteuert auf niedrigere Werte einstellen lässt.

Ein Problem bei eines derartigen, an sich wünschenswerten Elektrochirurgie-Generators ist, dass im Falle eines getakteten Wechselstroms keine zuverlässige Wirkleistungsbestimmung möglich ist, weil die Wirkleistungsbestimmungseinheit durch die Taktung des ausgegebenen Wechselstroms beeinträchtigt wird. Daher ist es im diesem Falle vorgesehen, dass die Wirkleistungsbestimmungseinheit ausgebildet ist, die von dem Tiefpassfilter generierte Gleichspannung mit einem vom Tastverhältnis des abgegebenen Wechselstroms abhängigen Korrekturwertes zu multiplizieren und eine derart korrigierte Gleichspannung als ein die Phasenverschiebung repräsentierendes Signal auszugeben.

Es versteht sich, dass eine derartige Korrektur der von dem Tiefpassfilter generierten Gleichspannung als das die Phasenverschiebung repräsentierende Signal auch unabhängig von einer erfindungsgemäßen Schalteinheit vorgesehen werden kann und somit einen auch selbstständig zu verwirklichenden Erfindungsgedanken darstellt. Mit anderen Worten ist es möglich, bei einem Elektrochirurgie-Generator der eingangs genannten Art eine vom Tastverhältnis abhängige Korrektur der Ausgangs-Gleichspannung des Tiefpassfilters vorzusehen, ohne dass der Elektrochirurgie-Generator solche Mittel wie die zuvor beschriebene Schalteinheit aufweist, die verhindern, dass dem Tiefpassfilter ein Detektorausgangsignal zugeführt wird, wenn der Hochspannungsgenerator abgeschaltet ist.

Die Kombination beider Maßnahmen - also Schalteinheit zum Sperren des Tiefpassfiltereingangs bei abgeschaltetem Hochspannungsgenerator und Korrektur der vom Tiefpassfilter generierten Gleichspannung in Abhängigkeit des Tastverhältnisses - führt zu einer deutlich zuverlässigeren Wirkleistungsbestimmung.,

Der Korrekturwert mit dem die von dem Tiefpassfilter erzeugte Gleichspannung zu multiplizieren ist, entspricht vorzugsweise dem Kehrwert des Tastverhältnisses.

Vorzugsweise weist der Elektrochirurgie-Generator eine Steuereinheit auf, die mit dem Hochspannungsgenerator verbunden ist und die diesen steuert. Die Steuereinheit ist vorzugsweise mit der Wirkleistungsbestimmungseinheit verbunden und ausgebildet, von der Wirkleistungsbestimmungseinheit ein Ausgangssignal zu empfangen, das von der Phasenverschiebung zwischen Strom und Spannung des von dem Elektrochirurgie-Generator im Betrieb abgegebenen, hochfrequenten Wechselstroms abhängt.

Vorzugsweise ist die Steuereinheit außerdem ausgebildet, an die Steuerschaltung ein das Tastverhältnis (D) repräsentierendes Signal abzugeben.

Die Erfindung soll anhand von Ausführungsbeispielsweisen mit Bezug auf die beigefügten Figuren näher erläutert werden. Von den Figuren zeigt:

Figur 1: Ein schematisches Blockschaltbild eines Elektrochirurgie-Generators mit angeschlossenem elektrochirurgischen Instrument;

Figur 2: ein schematisches Blockschaltbild einer Wirkleistungsbestimmungseinheit für einen Elektrochirurgie-Generator aus Figur 1;

Figur 3: ein beispielhaftes Schaltbild einer erfindungsgemäßen Wirkleistungsbestimmungseinheit.

Figur 4a und 4b: Skizze zur Erläuterung des Funktionsprinzips der Phasenverschiebungs-Bestimmungseinheit;

Figuren 5a bis c: Skizze von durch Nachschwingen des Hochspannungsgenerators beeinträchtigten Signalen zur Erläuterung des der Erfindung zugrunde liegenden Problems;

Figuren 6a bis c: Skizzen der Signale, wie sie ohne eine erfindungsgemäße Schalteinheit auftreten; und

Figuren 7a bis c: Skizzen der Signale mit einer erfindungsgemäßen Schalteinheit auftreten

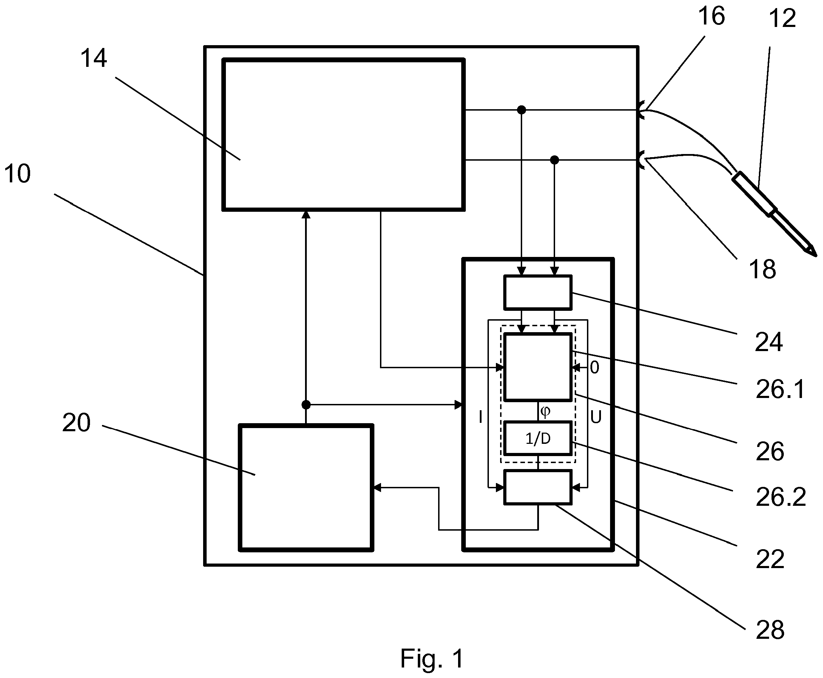

Figur 1 zeigt ein schematisches Blockschaltbild eines Elektrochirurgie-Generators 10 mit einem angeschlossenen chirurgischen Instrument 12.

Der Elektrochirurgie-Generator enthält einen Hochspannungsgenerator 14, der über seine Ausgänge 16 und 18 das Elektrochirurgie-Instrument 12 mit hochfrequenter Wechselspannung versorgt. Diese wird über das Elektrochirurgie-Instrument 12 an Körpergewebe abgegeben.

Um die über das Elektrochirurgie-Instrument 12 abgegebene Leistung zu steuern, ist die von dem Hochspannungsgenerator 14 abgegebene hochfrequente Wechselspannung moduliert, das heißt sie wird mit einem Tastverhältnis getaktet, indem der Hochspannungsgenerator 14 gemäß dem Tastverhältnis aus- und eingeschaltet wird. Das Ein- und Ausschalten des Hochspannungsgenerators 14 wird von einer Steuereinheit 20 gesteuert.

Die tatsächlich von dem Hochspannungsgenerator 14 über das Elektrochirurgie-Instrument 12 an Körpergewebe abgegebene Wirkleistung hängt dabei unter anderem auch von der Impedanz des Körpergewebes ab. Die Impedanz des Körpergewebes sowie andere Einflussgrößen bewirken eine Phasenverschiebung zwischen Spannung und Strom, das heißt zwischen der von dem Hochspannungsgenerator 14 abgegebenen hochfrequenten Wechselspannung und dem zugehörigen hochfrequenten Wechselstrom. Um die abgegebene Wirkleistung bestimmen zu können, muss der Elektrochirurgie-Generator 10 somit einen der jeweiligen aktuellen Phasenverschiebung repräsentierenden Wert bestimmen.

Hierzu ist typischerweise eine Wirkleistungsbestimmungseinheit 22 vorgesehen, die ausgebildet ist, eine Phasenverschiebung zwischen Strom und Spannung eines im Betrieb abgegebenen hochfrequenten Wechselstroms zu erfassen. Hierzu ist eine Strom- und Spannungsmesseinheit 24 elektrisch mit den Ausgängen 16 und 18 des Hochspannungsgenerators 14 verbunden und misst einerseits die jeweils momentane Spannung sowie andererseits den jeweils momentanen Strom und führt den Momentanwert des Stroms bzw. der Spannung repräsentierende Ausgangssignale einer Phasenverschiebung-Bestimmungseinheit 26 mit nicht gesondert dargestellten nachgeschaltetem Tiefpassfilter zu.

Da die Momentanwerte von Strom und Spannung variieren, repräsentieren die Eingangssignale der Phasenverschiebung-Bestimmungseinheit einerseits den zeitlichen Verlauf der von dem Hochspannungsgenerator 14 abgegebenen Spannung sowie andererseits den zeitlichen Verlauf des von dem Hochspannungsgenerator 14 abgegebenen Stroms. Das den zeitlichen Verlauf der Spannung repräsentierende Eingangssignal sowie das den zeitlichen Verlauf des Stroms repräsentierende Eingangssignal sind beide jeweils annähernd periodisch und zueinander phasenverschoben.

Die Phasenverschiebung-Bestimmungseinheit 26 ist ausgebildet, jeweils entweder positive oder negative Nulldurchgänge des Strom- bzw. Spannungsverlaufs zu erfassen und nach Erfassen des ersten Nulldurchgangs des Strom- und des Spannungsverlaufs solange ein Ausgangssignal mit positivem Betragswert auszugeben, bis ein entsprechender positiver oder negativer Nulldurchgang des zugehörigen, phasenverschobenen Strom- bzw. Spannungsverlaufs erfasst wird. Auf diese Weise ist ein von der Phasenverschiebung-Bestimmungseinheit erzeugtes Zwischenausgangssignal ein gepulstes Gleichspannungssignal, bei dem die jeweilige Pulsbreite zeitlich der Phasenverschiebung zwischen Strom und Spannung (oder umgekehrt) entspricht. Wenn dieses gepulste Gleichspannungssignal einem Tiefpassfilter zugeführt wird, ergibt sich ein geglättetes Gleichspannungssignal, dessen Betrag von der Phasenverschiebung $\varphi$ abhängt. Der Betrag ist größer, wenn die Phasenverschiebung größer ist und der Betrag des geglätteten (tiefpassgefilterten) Ausgangssignals ist kleiner, wenn die Phasenverschiebung kleiner ist. Das geglättete, tiefpassgefilterte Ausgangssignal der Phasenverschiebung-Bestimmungseinheit 26 repräsentiert somit die Phasenverschiebung zwischen Strom und Spannung und kann zur Bestimmung der von dem Hochspannungsgenerator 14 abgegebenen Wirkleistung herangezogen werden. Hierzu ist die Bestimmungseinheit 28 vorgesehen, die die Effektivwerte von Strom und Spannung bestimmt und aus diesen sowie der Phasenverschiebung die Wirkleitung ermittelt.

Da der die Phasenverschiebung repräsentierende geglättete Mittelwert auch vom Tastverhältnis abhängt, mit dem die von dem Hochspannungsgenerator 14 abgegebene hochfrequente Wechselspannung moduliert ist, wird der Wirkleistungsbestimmungseinheit auch ein das Tastverhältnis D repräsentierender Wert zugeführt. Hierzu ist die Wirkleistungsbestimmungseinheit 22 mit der Steuereinheit 20 verbunden. Umgekehrt wird der von der Wirkleistungsbestimmungseinheit 22 ermittelte Wert für die tatsächlich abgegebene Wirkleitung der Steuereinheit 20 zugeführt, um so eine Leistungsregelung zu ermöglichen.

Die Wirkleistungsbestimmungseinheit 22 kann dann die Wirkleitung wie folgt bestimmen:

$$P = U_{eff} \cdot I_{eff} \cdot \cos\left(\varphi \cdot \frac{1}{D}\right)$$

Die Phasenverschiebung-Bestimmungseinheit 26 enthält somit einen Teil 26.1, der den gleitenden Mittelwert der von der Phasenverschiebung abhängenden Impulsfolge 56 bildet, sowie einen zweiten Teil 26.2, mit dem dieser geglättete Mittelwert korrigiert wird. Die Korrektur, d.h. die Implementierung des zweiten Teils 26.2 sowie die Berechnung des Kosinus und der Wirkleistung kann in Software implementiert sein und in der Steuereinheit 20 erfolgen. D.h. die Bestandteile 26.2 und 28 können auch als Teil der Steuereinheit 20 in Software implementiert sein.

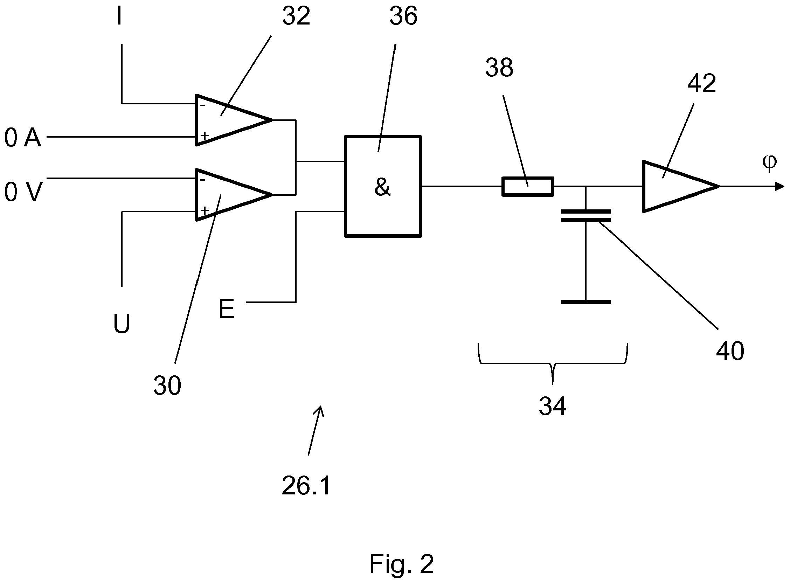

Eine mögliche Implementierung des ersten Teils 26.1 der Phasenverschiebung-Bestimmungseinheit 26 erfolgt als Schaltung und ist in Figur 2 abgebildet.

Ein erster Komparator 30 vergleicht einen jeweiligen Momentanwert der Spannung mit einem 0-Volt-Signal. Ein zweiter Komparator 32 vergleicht den jeweiligen Momentanwert des Stroms mit einem 0-Ampere-Signal. Solange der Momentanwert der Spannung größer als 0 Volt ist, gibt der erste Komparator 30 ein positives Ausgangssignal aus. Bei dem zweiten Komparator 32 wird der jeweilige Momentanwert des Stroms dem invertierten Eingang zugeführt, während das 0-Ampere-Signal dem nicht-invertierten Eingang des zweiten Komparators 32 zugeführt ist. Entsprechend gibt der zweite Komparator 32 solange ein negatives Ausgangssignal aus, solange der Momentanwert des Stroms größer ist, als 0 Ampere.

Solange somit sowohl der Momentanwert der Spannung als auch der Momentanwert des Stroms größer sind als der jeweilige Nullwert, heben sich die Ausgangswerte der beiden Komparatoren 30 und 32 auf, sodass die beiden Komparatoren 30 und 32 in der Summe ein Nullsignal ausgeben. Erst wenn der Momentanwert des Stroms kleiner wird als 0 Ampere, das heißt wenn der Zeitverlauf des Momentanwerts des Stroms einen Nulldurchgang in negative Richtung vollzieht, wird das kombinierte Ausgangssignal der beiden Komparatoren 30 und 32 positiv, und zwar solange, bis wenig später auch der zeitliche Verlauf des Momentanwerts der Spannung einen Nulldurchgang von positiver in negative Richtung vollzieht. In dem Augenblick, wo auch der Momentanwert der Spannung kleiner wird, als null Volt, und somit sowohl die Momentanwerte von Strom als auch Spannung beide jeweils kleiner sind als ein entsprechender Nullwert, heben sich die Ausgangssignale der Komparatoren 30 und 32 wieder auf. Dementsprechend ist der kombinierte Ausgangswert der beiden Komparatoren 30 und 32 nur solange verschieden, solange der Momentwert der Spannung schon kleiner ist, als null Volt, während der Momentanwert des Stroms noch größer ist als null Ampere. Auf diese Weise ist das kombinierte Ausgangssignal der beiden Komparatoren 30 und 32 ein gepulstes Gleichspannungssignal, dessen Pulsbreite genau der zeitlichen Dauer der Phasenverschiebung zwischen Strom und Spannungentspricht.

Die beiden Komparatoren 30 und 32 bilden somit einen Nulldurchgangsdetektor, dessen Ausgangssignal das gepulste Gleichspannungssignal ist.

Dieses gepulste Gleichspannungssignal wird einem Tiefpassfilter 34 zugeführt, welcher das gepulste Gleichspannungssignal glättet. Der Betrag des tiefpassgefilterten, geglätteten Gleichspannungssignals, das heißt also der Betrag des Ausgangssignals des Tiefpassfilters 34 hängt dabei von der Pulsbreite der Pulse des gepulsten, kombinierten Ausgangssignals der Komparatoren 30 und 32 ab. Je größer die Phasenverschiebung ist, umso größer ist die Pulsbreite des gepulsten Gleichspannungssignals und umso größer ist der Betrag des tiefpassgefilterten, geglätteten Ausgangssignals des Tiefpassfilters 34. Falls die Phasenverschiebung null ist, ist auch die Pulsbreite des gepulsten Gleichspannungssignals als kombiniertem Ausgangssignal der beiden Komparatoren 30 und 32 null und auch der Betrag des tiefpassgefilterten, geglätteten Ausgangssignals des Tiefpassfilters 34 ist null.

Da die von dem Hochspannungsgenerator 14 abgegebene Wirkleistung nicht nur von der Phasenverschiebung zwischen Strom und Spannung, sondern auch von dem Tastverhältnis abhängt, mit dem die hochfrequente Wechselspannung (bzw. der hochfrequente Wechselstrom) moduliert ist, spiegelt das tiefpassgefilterte, geglättete Ausgangssignal des Tiefpassfilters 34 nicht allein die Phasenverschiebung wider, sondern hängt auch von dem Tastverhältnis ab, mit dem die hochfrequente Ausgangsspannung des Hochspannungsgenerators 14 moduliert ist. Um tatsächlich einen nur die Phasenverschiebung repräsentierenden Wert zu erhalten, muss das geglättete, tiefpassgefilterte Ausgangssignal des Tiefpassfilters 34 somit noch mit dem Kehrwert des Tastverhältnisses D (also mit 1/D) multipliziert werden. Dies kann in der Steuereinheit 20 erfolgen.

In der Praxis führt auch dies nicht zu einem tatsächlich allein die Phasenverschiebung repräsentierenden Ausgangswert des Tiefpassfilters 34, weil ein Nachschwingen des Hochspannungsgenerators 14 nach dem Ausschalten dazu führt, dass die Phasenverschiebung-Bestimmungseinheit 26 auch dann noch Nulldurchgänge registriert und das kombinierte Ausgangssignal der Komparatoren 30 und 32 entsprechend Pulse der gepulsten Gleichspannung aufweist, wenn der Hochspannungsgenerator 14 eigentlich schon ausgeschaltet ist. Durch diese Nachschwinger wird der Ausgangswert des Tiefpassfilters 34 künstlich erhöht und suggeriert eine größere Phasenverschiebung, als tatsächlich vorhanden ist.

Um dieses Problem zu lösen, ist eine Schalteinheit 36 in Form eines UND-Gatters 36 vorgesehen, welches das kombinierte Ausgangssignal der Komparatoren 30 und 32 nur dann an den Tiefpassfilter 34 weitergibt, wenn der Hochspannungsgenerator 14 tatsächlich eingeschaltet ist. Dazu wird dem UND-Gatter 36 an einen Eingang das kombinierte Ausgangssignal der Komparatoren 30 und 32 zugeführt, also die gepulste Gleichspannung, während dem anderen Eingangssignal des UND-Gatters 36 das Einschaltsignal für den Hochspannungsgenerator 14 zugeführt wird. Nur wenn das Einschaltsignal positiv ist, das heißt der Hochspannungsgenerator 14 eingeschaltet ist, können somit die Pulse der gepulsten Gleichspannung als kombiniertes Ausgangssignal der Komparatoren 30 und 32 zum Ausgang des UND-Gatters 36 und somit zum Eingang des Tiefpassfilters 34 gelangen. Der Tiefpassfilter 34 empfängt somit nur solange das kombinierte Ausgangssignal der Komparatoren 30 und 32, solange der Hochspannungsgenerator 14 tatsächlich eingeschaltet ist, während ein etwaiges Ausschwingen ausgeblendet wird.

Wie Figur 2 zu entnehmen ist, sind die beiden Komparatoren 30 und 32 jeweils durch entsprechend geschaltete Operationsverstärker realisiert. Die Schalteinheit 36 ist, wie bereits ausgeführt, als UND-Gatter 36 ausgeführt. Der Tiefpassfilter 34 umfasst in an sich bekannter Weise einen Eingangswiderstand 38 und einen gegen Masse geschalteten Kondensator 40 sowie einen nachgeschalteten Operationsverstärker 42.

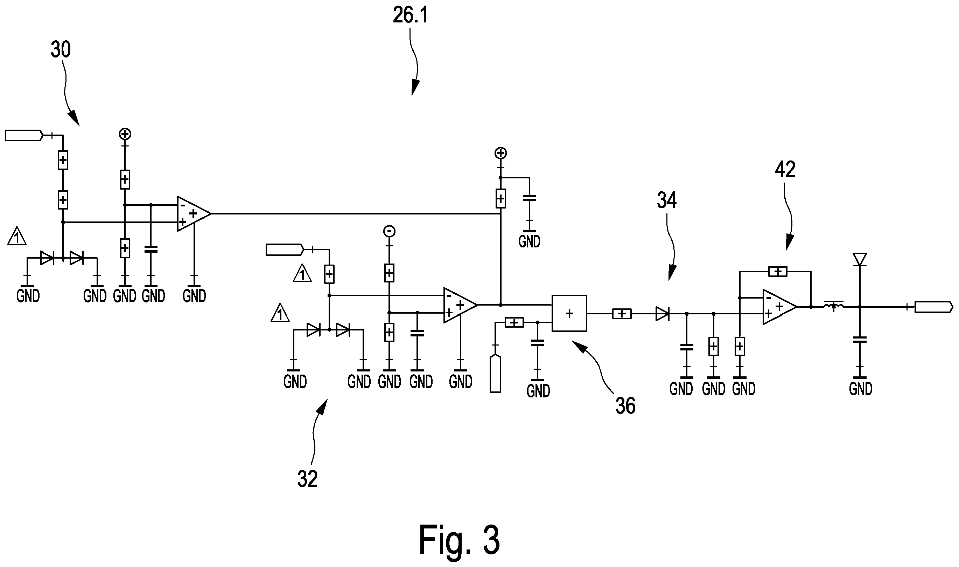

Figur 3 zeigt ein Beispiel für eine konkrete Ausgestaltung der Komparatoren 30, 32, des UND-Gatters 36 sowie des Tiefpassfilters 34 mit nachgeschaltetem Operationsverstärker.

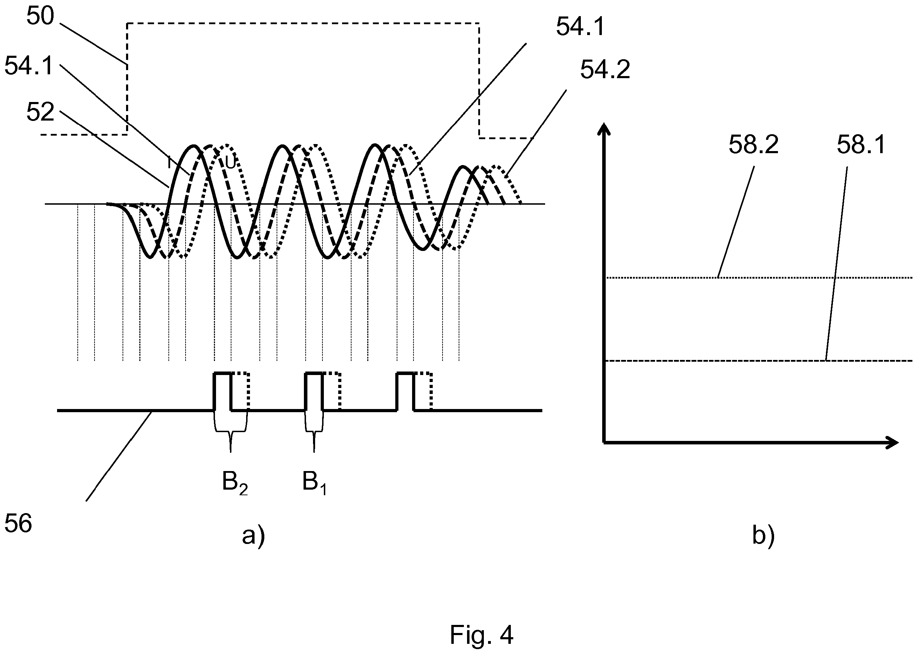

Figuren 4a und 4b illustrieren die Funktionsweise der Phasenverschiebungs-Bestimmungseinheit 26.

In Figur 4a ist in einer oberen Zeile gestrichelt der Verlauf des Einschaltsignals 50 für den Hochspannungsgenerator 14 dargestellt. Die durchgezogene Linie soll dabei den zeitlichen Verlauf des Stroms 52 repräsentieren, während die gestrichelte Linie den zeitlichen Verlauf der Spannung 54.1 beziehungsweise 54.2 repräsentiert. Der Einfachheit halber sind Strom und Spannung mit der gleichen Amplitude dargestellt, wobei in der Praxis die Amplituden - auch abhängig von der Skalierung unterschiedlich sein können. In Figur 4a sind zwei unterschiedliche Spannungsverläufe 54.1 und 54.2 dargestellt, die zwei unterschiedliche Phasenverschiebungen zwischen Strom und Spannung repräsentieren. Im Falle des Spannungsverlaufs 54.1 ist die Phasenverschiebung zwischen Strom und Spannung kleiner, als im Falle des Spannungsverlaufs 54.2. Die untere Zeile in Figur 4 repräsentiert das kombinierte Ausgangssignal 56 der Komparatoren 30 und 32. Wie zu erkennen ist, ist das kombinierte Ausgangssignal 56 ein gepulstes Gleichspannungssignal bei dem die Pulsbreite B genau der Dauer der Phasenverschiebung zwischen Strom und Spannung entspricht. Im Falle der kleineren Phasenverschiebung zwischen dem Stromverlauf 52 und dem Spannungsverlauf 54.1 haben die Pulse des gepulsten Gleichspannungssignals 56 eine Dauer B1. Im Falle einer größeren Phasenverschiebung ist die Pulsdauer länger und nimmt den Wert B2 an.

Wenn die gepulste Gleichspannung, die in der unteren Zeile in Figur 4a dargestellt ist, tiefpassgefiltert und dadurch geglättet wird, ergibt sich eine Gleichspannung mit einem Betrag, der von der Dauer, d. h. der Pulsbreite der Pulse des gepulsten Gleichspannungssignals 56 abhängt. Dies ist in Figur 4b dargestellt. Die gestrichelte untere Linie 58.1 repräsentiert eine tiefpassgefilterte Gleichspannung wie sie durch Tiefpassfiltern der gepulsten Gleichspannung mit der Pulsbreite B1 erzielt wird, während die obere, gepunktete Linie 58.2 eine geglättete Gleichspannung mit einem höheren Betrag darstellt, wie sie durch Tiefpassfiltern der gepulsten Gleichspannung 56 mit Spannungspulsen einer Dauer B2 erzielt wird.

Figur 4a zeigt der Einfachheit halber ausschnittsweise einen annähernd eingeschwungenen Zustand bei eingeschaltetem Hochspannungsgenerator 14.

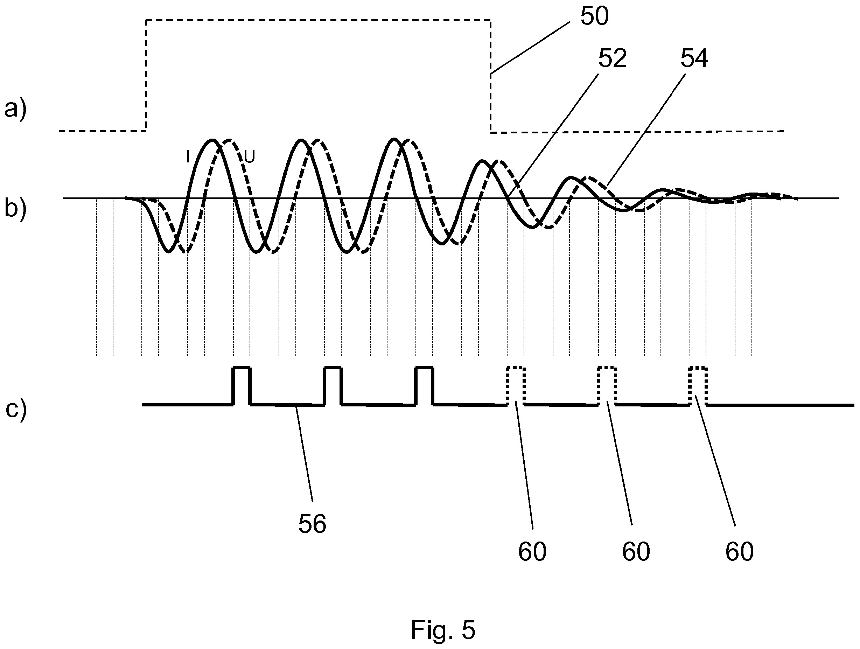

Figur 5 zeigt, dass nach Ausschalten des Hochspannungsgenerators 14 - erkennbar am Abfall des Einschaltsignals 50; siehe Figur 5a) - noch Nachschwingungen im Stromverlauf 52 und dem Spannungsverlauf 54 auftreten (siehe Figur 5b), die zu weiteren registrierten Nulldurchgängen und damit zu dem Pulsen 60 im Verlauf des gepulsten Gleichspannungssignals 56 führen (siehe Figur 5c). Wird das gepulste Gleichspannungssignal 56 über einen längeren Zeitraum tiefpassgefiltert, hängt der Betrag der sich ergebenden, geglätteten Gleichspannungssignals auch von den durch Nachschwinger verursachten Pulsen 60 ab. Wären diese nicht vorhanden, wäre der Betrag des tiefpassgefilterten, geglätteten Gleichspannungssignals niedriger. Wenn der Betrag des geglätteten Gleichspannungssignals mit dem Kehrwert des Tastverhältnisses für das Einschaltsignal 50 multipliziert wird, ergibt sich aufgrund der durch Nachschwinger verursachten Pulse 60 ein Betrag und damit ein Ausgangssignal der Phasenverschiebung-Bestimmungseinheit, das die Phasenverschiebung zwischen Strom und Spannung nicht korrekt wiedergibt, sondern durch die auf Nachschwingen beruhenden Pulse 60 verfälscht ist.

Um dem entgegenzuwirken, ist bei der Phasenverschiebungs-Bestimmungseinheit 26 die Schalteinheit 36, nämlich das UND-Gatter 36 vorgesehen, welches die durch Nachschwingen verursachten Pulse 60 de facto ausblendet.

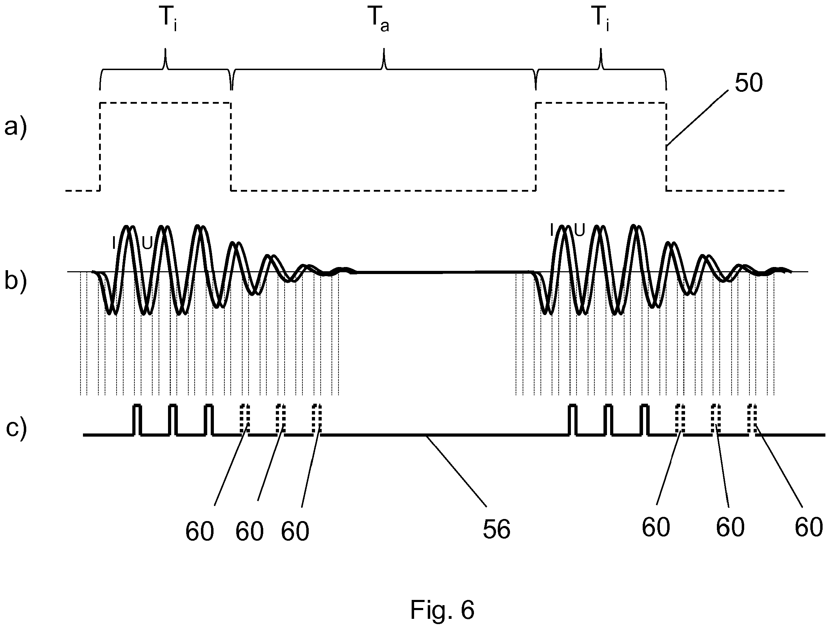

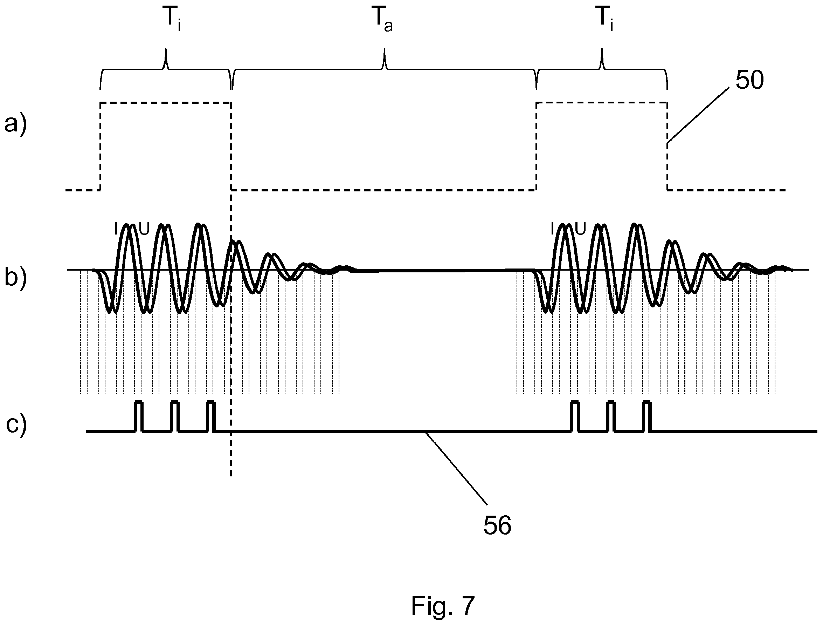

Dies ist in den Figuren 6 und 7 erläutert. Figur 6 zeigt den Verlauf der gepulsten Gleichspannung 56, wie er sich ergibt, wenn keine Schalteinheit 36 vorgesehen ist.

Figur 7 zeigt, dass die Pulse 60 durch die Schalteinheit 36 ausgeblendet sind. Wird das in Figur 7 in der unteren Zeile abgebildete gepulste Gleichspannungssignal 56 tiefpassgefiltert und der tiefpassgefilterte Wert mit dem Kehrwert des Tastverhältnisses des Einschaltsignals 50 multipliziert, spiegelt der sich ergebene Betrag die Phasenverschiebung zwischen Stromverlauf 52 und Spannungsverlauf 54 recht genau wider.

Das Tastverhältnis des Einschaltsignals 50 ist dabei

$$D = \frac{T_i}{T_a} .$$

Der Kehrwert des Tastverhältnisses ist somit

$$\frac{1}{D} = \frac{T_a}{T_i} .$$

**Patentansprüche**

1. Elektrochirurgie-Generator (10) mit Anschlüssen (16, 18) für ein elektrochirurgisches Instrument und mit einem Hochspannungsgenerator (14), der mit den Anschlüssen (16, 18) elektrisch verbunden und ausgebildet ist, in seinem eingeschalteten Zustand einen hochfrequenten Wechselstrom zu erzeugen und über die Anschlüsse (16, 18) abzugeben, wobei der Elektrochirurgie-Generator (10) eine Wirkleistungsbestimmungseinheit (22) aufweist, die eine Phasenverschiebung-Bestimmungseinheit (26) umfasst, welche ausgebildet ist, ein Ausgangssignal (58) zu liefern, dass eine Phasenverschiebung zwischen Strom und Spannung eines im Betrieb abgegebenen Wechselstroms repräsentiert, wobei die Phasenverschiebung-Bestimmungseinheit (26) ausgebildet ist, ein gepulstes Gleichspannungssignal (56) zu erzeugen, bei dem die Pulsbreite (B) eine zeitliche Differenz zwischen den Nulldurchgängen von Strom und Spannung - und damit die Phasenverschiebung - widerspiegelt, und das gepulste Gleichspannungssignal (56) mittels eines Tiefpassfilters (34) zu einem Tiefpassfilter-Ausgangssignal zu verarbeiten, dessen Betrag von der Pulsbreite (B) der Pulse des gepulsten Gleichspannungssignals (56) abhängt, **dadurch gekennzeichnet, dass** eine Schalteinheit (36) vorgesehen ist, die derart angeordnet und ausgebildet ist, dass dem Tiefpassfilter (34) nur dann die gepulste Gleichspannung zugeführt wird, wenn der Hochspannungsgenerator (14) eingeschaltet ist, nicht aber, wenn der Hochspannungsgenerator (14) ausgeschaltet ist.

2. Elektrochirurgie-Generator (10) nach Anspruch 1, **dadurch gekennzeichnet, dass** Phasenverschiebung-Bestimmungseinheit (26) einen Nulldurchgangdetektor (30, 32) aufweist, der die gepulste Gleichspannung (56) in Abhängigkeit der Phasenverschiebung erzeugt, und die Schalteinheit (36) zwischen einem Ausgang des Nulldurchgangdetektors und einem Eingang des Tiefpassfilters (34) angeordnet ist.

3. Elektrochirurgie-Generator (10) nach Anspruch 1 oder 2, **dadurch gekennzeichnet, dass** die Schalteinheit als UND-Gatter (36) realisiert ist, dessen einer Eingang mit einem Ausgang der Phasenverschiebung-Bestimmungseinheit (26) verbunden ist und dessen anderer Eingang im eingeschaltetem Zustand des Hochspannungsgenerators (14) ein Generator-Ein-Signal empfängt, und dessen Ausgang mit dem Tiefpassfilter (34) verbunden ist.

4. Elektrochirurgie-Generator (10) nach Anspruch 2, **dadurch gekennzeichnet, dass** der Nulldurchgangdetektor zwei Komparatoren (30, 32) aufweist, von denen ein erster Komparator (30) ausgebildet und angeordnet ist, ein Spannungssignal mit einem Nullsignal zu vergleichen und ein Ausgangsignal zu erzeugen, wenn ein Momentanwert der Spannung größer ist, als das Nullsignal, während der zweite Komparator (32) ausgebildet und angeordnet ist, ein Nullsignal mit einem Stromsignal zu vergleichen und ein Ausgangsignal zu erzeugen, wenn das Nullsignal größer ist als ein Momentanwert des Stroms, wobei die Ausgänge der Komparatoren miteinander verbunden sind, sodass die beiden Komparatoren nur dann ein kombiniertes Ausgangssignal als Nulldurchgangsdetektor-Ausgangssignal (56) mit einem über einem Schwellwert liegenden Betrag ausgeben, wenn der Momentanwert der Spannung positiv (also größer als das Nullsignal) ist und gleichzeitig der Momentanwert des Stroms negativ (also kleiner als das Nullsignal) ist; oder umgekehrt.

5. Elektrochirurgie-Generator (10) nach wenigstens einem der Ansprüche 1 bis 4, **dadurch gekennzeichnet, dass** der Hochspannungsgenerator (14) ausgebildet ist, in seinem Betriebszustand den hochfrequenten Wechselstrom mit einem Tastverhältnis D getaktet über die Anschlüsse (16, 18) abzugeben **dadurch gekennzeichnet, dass** die Wirkleistungsbestimmungseinheit (22) ausgebildet ist, die Gleichspannung mit einem vom Tastverhältnis D abhängigen Korrekturwert $\dfrac{1}{D}$ zu multiplizieren und eine korrigierte Gleichspannung als ein die Phasenverschiebung repräsentierendes Signal auszugeben.

6. Elektrochirurgie-Generator (10) nach Anspruch 5, **dadurch gekennzeichnet, dass** der Korrekturwert dem Kehrwert

$\dfrac{1}{D}$ des Tastverhältnisses D entspricht.

7. Elektrochirurgie-Generator (10) nach wenigstens einem der Ansprüche 1 bis 6, **dadurch gekennzeichnet, dass** der Elektrochirurgie-Generator (10) eine Steuereinheit (20) aufweist, die mit dem Hochspannungsgenerator (14) verbunden ist und die diesen im Betrieb steuert.

8. Elektrochirurgie-Generator (10) nach Anspruch 7, **dadurch gekennzeichnet, dass** die Steuereinheit (20) mit der Wirkleistungsbestimmungseinheit (22) verbunden und ausgebildet ist, von der Wirkleistungsbestimmungseinheit (22) ein Ausgangssignal zu empfangen, das von der Phasenverschiebung zwischen Strom und Spannung des von dem Elektrochirurgie-Generator (10) im Betrieb abgegebenen, hochfrequenten Wechselstroms abhängt.

9. Elektrochirurgie-Generator (10) nach Anspruch 7 oder 8, **dadurch gekennzeichnet, dass** die Steuereinheit (20) mit der Wirkleistungsbestimmungseinheit (22) verbunden und ausgebildet ist, an die Steuerschaltung ein das Tastverhältnis D repräsentierendes Signal abzugeben.

**Claims**

1. An electrosurgical generator (10) having connectors (16,18) for an electrosurgical instrument and comprising a high-voltage generator (14), which is electrically connected to the connectors (16,18) and which is designed to produce a high-frequency alternating current in its activated state and output said high-frequency alternating current via the connectors (16, 18), wherein the electrosurgical generator (10) has an effective power determination unit (22) comprising a phase shift determination unit (26) that is designed to supply an output signal (58) representing a phase shift between the current and the voltage of an alternating current output during operation, wherein the phase shift determination unit (26) is designed to produce a pulsed DC voltage signal (56), in which the pulse width (B) reflects a time difference between the zero crossings of the current and the voltage - and consequently the phase shift - and to process the pulsed DC voltage signal (56) by means of a low-pass filter (34) to form a low-pass filter output signal, the magnitude of which depends on the pulse width (B) of the pulses of the pulsed DC voltage signal (56), **characterized in that** a switch unit (36) is provided which is arranged and designed to ensure that the low-pass filter (34) is only supplied with the pulsed DC voltage if the high-voltage generator (14) is switched on, but not if the high-voltage generator (14) is switched off.

2. An electrosurgical generator (10) pursuant to claim 1, **characterized in that** the phase shift determination unit (26) has a zero crossing detector (30, 32) that produces the pulsed DC voltage (56) in dependence on the phase shift, and **in that** the switch unit (36) is arranged between an output of the zero crossing detector and an input of the low-pass filter (34).

3. An electrosurgical generator (10) pursuant to claim 1 or 2, **characterized in that** the switch unit is realized as an AND gate (36), one of the inputs of which is connected to an output of the phase shift determination unit (26) and the other input of which receives, when the high-voltage generator (14) is in its activated state, a "generator on" signal, and the output of which is connected to the low-pass filter (34).

4. An electrosurgical generator (10) pursuant to claim 2, **characterized in that** the zero crossing detector has two comparators (30, 32), of which a first comparator (30) is designed and arranged to compare a voltage signal with a zero signal and to produce an output signal if an instantaneous value of the voltage is greater than the zero signal, while the second comparator (32) is designed and arranged to compare a zero signal with a current signal and to produce an output signal if the zero signal is greater than the instantaneous value of the current, wherein the outputs of the comparators are connected to each other so that the two comparators will only output a combined output signal as a zero crossing detector output signal (56) with a magnitude exceeding a threshold value, if the instantaneous value of the voltage is positive (i.e. greater than the zero signal) and if, at the same time, the instantaneous value of the current is negative (i.e. less than the zero signal); or vice versa.

5. An electrosurgical generator (10) pursuant to at least one of claims 1 to 4, **characterized in that** the high-voltage generator (14) is designed to output, in its activated state, the high-frequency alternating current pulsed with a duty cycle D via the connectors (16, 18)
**characterized in that** the effective power determination unit (22) is designed to multiply the

DC voltage by a correction value $\dfrac{1}{D}$ dependent on the duty cycle D and to output a corrected DC voltage as a signal representing the phase shift.

6. An electrosurgical generator (10) pursuant to claim 5, **characterized in that** the correction value corresponds to the reciprocal $\dfrac{1}{D}$ of the duty cycle D.

7. An electrosurgical generator (10) pursuant to at least one of claims 1 to 6, **characterized in that** the electrosurgical generator (10) has a control unit (20) that is connected to the high-voltage generator (14) and controls the high-voltage generator (14) during operation.

8. An electrosurgical generator (10) pursuant to claim 7, **characterized in that** the control unit (20) is connected to the effective power determination unit (22) and designed to receive an output signal from the effective power determination unit (22), which depends on the phase shift between the current and the voltage of the high-frequency alternating current output by the electrosurgical generator (10) during operation.

9. An electrosurgical generator (10) pursuant to claim 7 or 8, **characterized in that** the control unit (20) is connected to the effective power determination unit (22) and designed to output a signal representing the duty cycle D to the control circuit.

**Revendications**

1. Générateur (10) d'électrochirurgie ayant des bornes (16, 18) pour un instrument électrochirurgical et ayant un générateur (14) de haute tension, qui est relié électriquement aux bornes (16, 18) et qui est constitué pour, dans son état mis en circuit, produire et fournir, par les bornes (16, 18), un courant alternatif de haute fréquence, le générateur (10) d'électrochirurgie ayant une unité (22) de détermination de la puissance active, qui comprend une unité (26) de détermination du déphasage, laquelle est constituée pour fournir un signal (58) de sortie, qui représente un déphasage entre courant et tension d'un courant alternatif fourni en fonctionnement, l'unité (26) de détermination de déphasage étant constituée pour produire un signal (56) de tension continue puisée, dans lequel la largeur (B) d'impulsion reflète une différence dans le temps entre les passages par zéro du courant et de la tension - et ainsi le déphasage - et pour transformer le signal (56) de tension continue puisée, au moyen d'un filtre (34) passe-bas, en un signal de sortie de filtre passe-bas, dont la valeur dépend de la largeur (B) des impulsions du signal (56) de tension continue puisée,
**caractérisé en ce qu'**il est prévu une unité (36) de coupure, qui est montée et constituée de manière à n'appliquer, au filtre (34) passe-bas, la tension continue puisée, que si le générateur (14) de haute tension est mis en circuit, mais non si le générateur (14) de haute tension est mis hors circuit.

2. Générateur (10) d'électrochirurgie suivant la revendication 1, **caractérisé en ce que** l'unité (26) de détermination de déphasage a un détecteur (30, 32) de passage au point zéro, qui produit la tension (56) continue pulsée en fonction du déphasage et l'unité (36) de coupure est montée entre une sortie du détecteur de passage au point zéro et une entrée du filtre (34) passe-bas.

3. Générateur (10) d'électrochirurgie suivant la revendication 1 ou 2, **caractérisé en ce que** l'unité de coupure est réalisée en porte (36) ET, dont une entrée est reliée à une sortie de l'unité (26) de détermination du déphasage et dont l'autre entrée reçoit, lorsque le générateur (14) de haute tension est à l'état mis en circuit, un signal d'entrée de générateur, et dont la sortie est reliée au filtre (34) passe-bas.

4. Générateur (10) d'électrochirurgie suivant la revendication 2, **caractérisé en ce que** le détecteur de passage par zéro a deux comparateurs (30, 32), dont un premier comparateur (30) est constitué et monté de manière à comparer un signal de tension à un signal de zéro et à produire un signal de sortie, si une valeur instantanée de la tension est plus haute que le signal de zéro, tandis que le deuxième comparateur (32) est constitué et monté de manière à comparer un signal de zéro à un signal de courant et à produire un signal de sortie, si le signal de zéro est plus grand qu'une valeur instantanée du courant, les sorties des comparateurs étant reliées entre elles, de manière à ce que les deux comparateurs ne fournissent un signal de sortie combiné, comme signal (56) de sortie de détecteur de passage par zéro ayant une valeur au-dessus d'une valeur de seuil, que si la valeur instantanée de la tension est positive (donc plus haute que la valeur zéro) et si en même temps la valeur instantanée du courant est négative (donc plus petite que la valeur zéro) ; ou inversement.

5. Générateur (10) d'électrochirurgie suivant au moins l'une des revendications 1 à 4, **caractérisé en ce que** le générateur (14) de haute tension est constitué pour fournir, par l'intermédiaire des bornes (16, 18), dans son état de fonctionnement, le courant alternatif de haute fréquence cadencé à un rapport cyclique D,
**caractérisé en ce que** l'unité (22) de détermination de puissance active est constituée pour multiplier la tension continue par une valeur 1/D de correction, qui dépend du rapport cyclique D, et pour fournir une tension continue corrigée, comme signal représentant le déphasage.

6. Générateur (10) d'électrochirurgie suivant la revendication 5, **caractérisé en ce que** la valeur de correction correspond à l'inverse 1/D du rapport cyclique D.

7. Générateur (10) d'électrochirurgie suivant au moins l'une des revendications 1 à 6, **caractérisé en ce que** le générateur (10) d'électrochirurgie a une unité (20) de commande, qui est reliée au générateur (14) de haute tension et qui commande celui-ci en fonctionnement.

8. Générateur (10) d'électrochirurgie suivant la revendication 7, **caractérisé en ce que** l'unité (20) de commande est reliée à l'unité (22) de détermination de puissance active et est constituée pour recevoir, de l'unité (22) de détermination de puissance active, un signal de sortie, qui dépend du déphasage entre courant et tension du courant alternatif de haute fréquence fourni par le générateur (10) d'électrochirurgie en fonctionnement.

9. Générateur (10) d'électrochirurgie suivant la revendication 7 ou 8, **caractérisé en ce que** l'unité (20) de commande est reliée à l'unité (22) de détermination de puissance active et est constituée pour fournir au circuit de commande un signal représentant le rapport cyclique D.

Fig. 1

EP 3 634 283 B1

Fig. 2

Fig. 3

Fig. 4

a)

b)

c)

Fig. 5

EP 3 634 283 B1

Fig. 6

EP 3 634 283 B1

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011078452 A1
- DE 102010040824 A1
- WO 9844855 A1
- DE 102007051097 A1